# EUROPEAN PATENT SPECIFICATION

(11) **EP 3 709 941 B1**
(45) Date of publication and mention of the grant of the patent: **14.12.2022**
(21) Application number: 18812313.7
(22) Date of filing: 18.11.2018
(51) Int. Cl.: A61F 9/007

(54) **HANDHELD IMPLANTATION DEVICES FOR IMPLANTATION OF RETINAL TISSUE IMPLANT**
TRAGBARE IMPLANTATIONSVORRICHTUNGEN ZUR IMPLANTATION VON RETINAGEWEBEIMPLANTATEN
DISPOSITIFS D'IMPLANTATION PORTATIFS POUR IMPLANTATION D'UN IMPLANT DE TISSU RÉTINIEN

(30) Priority: 21.11.2017 IL 25579617
(43) Date of publication of application: 23.09.2020
(73) Proprietor: E.K. - D.D.S. Ltd, 6706038 Tel Aviv (IL)
(72) Inventor: BEN NUN, Joshua, 40291 Beit Herut (IL)
(74) Representative: South, Nicholas Geoffrey
(86) International application number: PCT/IL2018/051241
(87) International publication number: WO 2019/102455

(56) References cited:
- WO-A1-00/76403
- WO-A1-94/21205
- WO-A1-2015/111040
- WO-A2-2012/149468

## Description

### Field of the Invention

The invention relates to handheld implantation devices for implantation of retinal tissue implants.

### Background of the Invention

The human retina, as in many other vertebrates, has evolved as a layered configuration with an interior retina constituting a neuronal component and an outer retina constituting a light sensing component. The light sensing component is further layered and includes a monolayer of specialized cells known as Retinal Pigment Epithelium (RPE) and a photoreceptor layer. Light entering a human eye passes through a transparent neuronal component before being captured by the photoreceptor layer, transformed to nerve stimuli and sent "backwards" to the neuronal component and from there to the brain. The light sensing component includes over 150 million photoreceptors. The neuronal component organizes the enormous stimuli input to a coded message and converges it to 1.5 million ganglion cells that transfer the coded message to the brain via the optic nerve which is a bundle of all the axons originating from the ganglion cells - one axon per each ganglion cell. The human fovea includes about half of the total number of photoreceptors and is responsible for visual acuity.

Transformation of light photons to nerve stimuli involves biochemical reactions generating large amount of biological waste that must be removed instantaneously and efficiently to maintain uninterrupted visual function. The waste removal is performed by the Retinal Pigment Epithelium (RPE) cells. The apexes of these cells are physically interdigitating with the leading active surfaces of the photoreceptors where the photochemical reactions occur enabling instantaneous removal of the consumed photoreceptor tips and exposure of the segments behind them for further reaction.

Some blinding disorders are caused by pathologic processes largely limited to the outer retina. Such disorders include Age-Related Macular Degeneration (ARMD or AMD) and Retinitis Pigmentosa (RP). Since an inner retina remains functional long after disappearance of a damaged outer retina, vision recovery seems possible by stimulation of a survived inner retina. The concept has been confirmed using electrodes that stimulate an inner retina with electrical pulses. However, the ability to create a sustainable electro-biological interface at the level of organization required for reasonable vision is beyond current knowledge and technology. It was logical to attempt to implant a retinal graft of normal outer-retinal tissue taken from a human cadaver eye or laboratory cultivated from stem cells. Clinical studies conducted over the last decade demonstrate the feasibility of using viable tissue implantation to treat outer retina blinding disorders by encouraging results. However, regardless of the composition of an outer-retinal tissue implant be it human embryonic retinal tissue or laboratory cultivated outer retinal layer tissue, the transfer of such a delicate implant to a human eye has been found to be a major obstacle further emphasized by recent findings that the accurate and stable interface between an outer-retinal tissue implant and a recipient inner retina is a critical factor for successful implantation.

WO 2015/111040 A1 discloses a tissue holding assembly for endothelial implantation comprising an open frame having legs for engaging a stem, such that said frame is insertable between a stroma and a Descemet's membrane, said frame being coated with a biological adhesive for adhering to a perimeter of a section of the Descemet's membrane to adhere the section of the Descemet's membrane to the frame for surgical separation of said section by cutting therearound, wherein said frame further comprises a heating element around its inner perimeter.

WO 2012/149468 A2 discloses instruments and methods for delivery of substrates, including cell- seeded substrates, to target tissues.

WO 94/21205 A1 discloses a method and surgical instrument for transplanting a graft in the subretinal area of a host eye.

WO 00/76403 A1 discloses an instrument for implanting retinal tissue into the subretinal space of the eye.

### Summary of the Invention

The present invention is directed toward handheld implantation devices for implantation of retinal tissue implants and in particular outer-retinal tissue implants. The invention provides a handheld implantation device according to claim 1. The retinal tissue implant can be either full thickness embryonic tissue or partial thickness or full thickness laboratory cultivated tissue. The handheld implantation devices include an elongated handheld implantation tool, an implant holder for peripherally holding a retinal tissue implant, and a clinician-operated attachment arrangement for initial attaching the implant holder to the implantation tool and subsequent detaching the implant holder together with its retinal tissue implant therefrom at an implantation site. Different attachment arrangements can be employed for detachably attaching an implant holder to a handheld implantation tool. In the case of an outer-retinal tissue implant, an implant holder is preferably oval shaped having a major axis between about 6 mm to about 8 mm and a minor axis between about 3 mm to about 5 mm and is intended to be implanted such that its center is implanted at a human eye's fovea.

### Brief Description of Drawings

In order to understand the invention and to see how it can be carried out in practice, preferred embodiments will now be described, by way of nonlimiting examples only, with reference to the accompanying drawings in which similar parts are likewise numbered, and in which:
Fig. 1A is a front perspective view of an assembled handheld implantation device including a handheld implantation tool and a detachable implant holder peripherally holding an outer-retinal tissue implant in accordance with a first embodiment of the present invention;
Fig. 1B is a longitudinal cross section of the implantation tool along line 1B-1B in Figure 1A;
Fig. 1C is a transverse cross section of the implantation tool along line 1C-1C in Figure 1B;
Fig. 2A is a front perspective view of the implantation tool and the implant holder subsequent to detachment of the implant holder therefrom;
Fig. 2B is a longitudinal cross section of the implantation tool along line 2B-2B in Figure 2A;
Fig. 3 is a top plan view of an implant carrier of the implant holder;
Fig. 4 is a front elevation view of the implant carrier of the implant holder;
Fig. 5 is an exploded view of the implant holder including its implant carrier, its outer-retinal tissue implant and its implant carrier surround;
Fig. 6 is a transverse cross section showing an initial step in the preparation of the implant holder;
Fig. 7 is a transverse cross section showing the implant holder before trimming the downward depending edge of its outer-retina tissue implant;
Fig. 8 is a transverse cross section of the implant holder along line 8-8 in Figure 1A;
Fig. 9 is a front perspective view of a pre-assembled handheld implantation device including a handheld implantation tool and a detachable implant holder in accordance with a second embodiment of the present invention;
Fig. 10 is a top plan view of an implant carrier of the Figure 9 implant holder;
Fig. 11 is a bottom plan view of the implant carrier of the Figure 9 implant holder;
Fig. 12 is a front elevation view of the implant carrier of the Figure 9 implant holder;
Fig. 13 is a front perspective view of a leading implantation tool end of the implantation tool on attachment of the implant holder thereto; and
Fig. 14 is a front perspective view of the leading implantation tool end of the implantation tool after detachment of the implant holder therefrom.

### Detailed Description of Drawings

The present invention is described for implanting an outer-retinal tissue implant hereinafter abbreviated as ORTI but can be equally used for implanting other retinal tissue implants. Retinal tissue implants can have a thickness T from between about 15 microns to about 250 microns depending on their intended implantation site, intended therapeutic action, and the like.

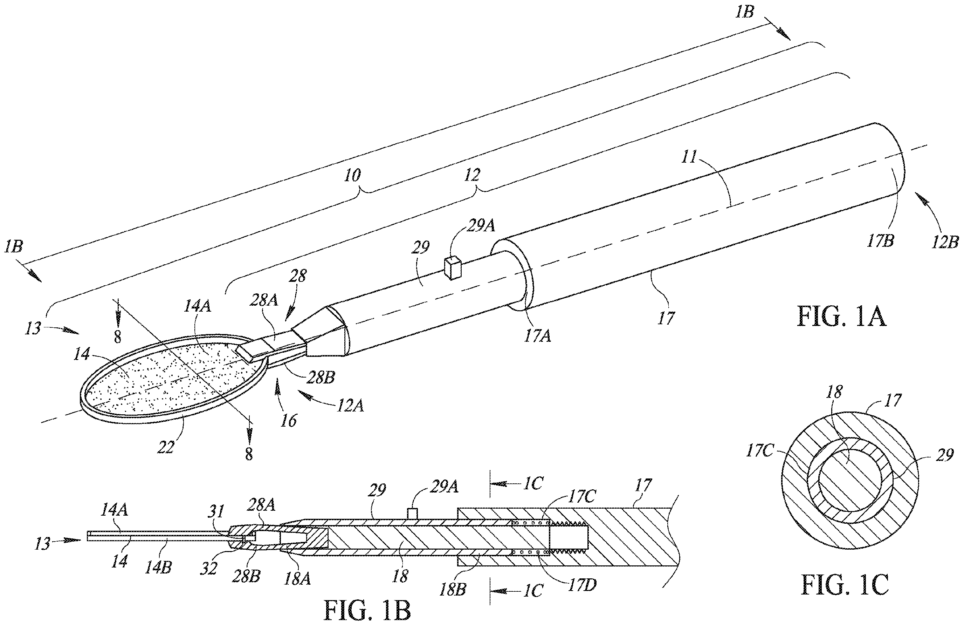

Figure 1 to Figure 8 show a handheld implantation device 10 has a longitudinal implantation device centerline 11 and includes a handheld implantation tool 12, a generally planar implant holder 13 peripherally holding an ORTI 14 and a releasable clamping arrangement 16 constituting a clinician-operated attachment arrangement for attaching the implant holder 13 to the implantation tool 12 for enabling its selective detachment therefrom together with the ORTI 14 at an implantation site. The handheld implantation tool 12 has a leading implantation tool end 12A and a trailing implantation tool end 12B. The ORTI 14 has uppermost viable retinal tissue 14A and a lowermost basement membrane 14B. The handheld implantation tool 12 includes a handle 17 having a leading handle end 17A and a trailing handle end 17B. The handle 17 has a blind recess 17C extending rearwards from the leading handle end 17A towards the trailing handle end 17B. The blind recess 17C has a transverse circular cross section. The handle 17 supports a shaft 18 inserted into the blind recess 17C for insertion into a recipient's eye through an incision formed in the eye's scleral wall at the pars plana. The shaft 18 has a transverse circular cross section, a leading shaft end 18A and a trailing shaft end 18B at the base of the blind recess 17C. The trailing shaft end 18B is screw threaded into the handle 17 or similarly attached thereto.

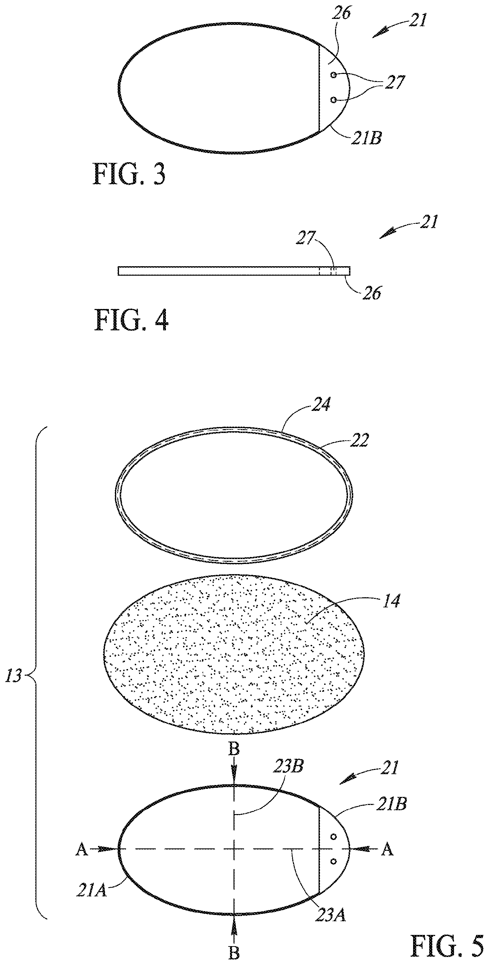

The implant holder 13 preferably has an oval shape for implanting an ORTI but equally can have other shapes suitable for peripherally holding an ORTI. Accordingly, the implant holder 13 has a major axis 19A co-axial with the longitudinal implantation device centerline 11 defining a leading implant holder end 13A remote from the handheld implantation tool 12 and a trailing implant holder end 13B adjacent the handheld implantation tool 12, and a minor axis 19B. The implant holder 13 includes an implant carrier 21 and a closed implant carrier surround 22 for snugly peripherally mounting on the implant carrier 21 for peripherally holding the ORTI 14 thereon. The implant carrier 21 is manufactured from suitable biocompatible load bearing material, for example, nitinol and the like, having shape memory properties. The implant carrier 21 has a leading implant carrier end 21A and a trailing implant carrier end 21B. The implant carrier 21 is resiliently flexible about a major axis 23A and a minor axis 23B as correspondingly denoted by arrows A and B (see Figure 5). The implant carrier surround 22 is manufactured from suitable biocompatible resiliently flexible material, for example, silicon, and the like. The implant carrier surround 22 has a generally C-shaped transverse cross section with an internal implant carrier surround groove 24 intended to snugly receive the implant carrier 21 therein thereby peripherally entrapping the ORTI 14. The implant carrier 21 has a segment-like carrier plate 26 at its trailing implant carrier end 21B. The segment-like carrier plate 26 is preferably parallel to the minor axis 23B and distanced therefrom to occupy a relatively small area of the implant carrier 21. The segment-like carrier plate 26 has a spaced apart pair of clamping throughgoing bores 27.

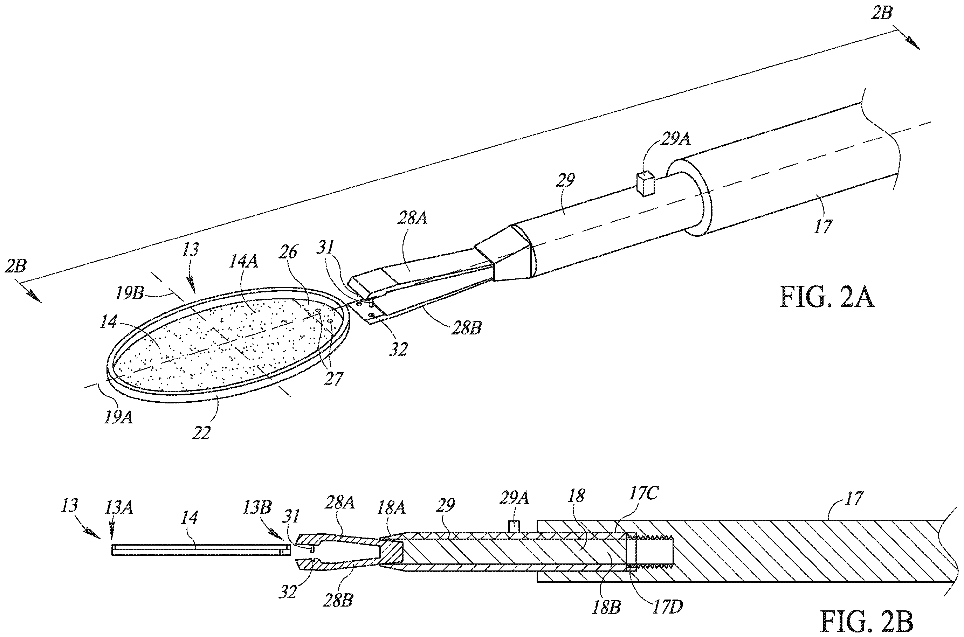

The implantation tool 12 includes a normal open clamping jaw pair 28 at the leading shaft end 18A. The normal open clamping jaw pair 28 includes an uppermost clamping jaw 28A and a lowermost clamping jaw 28B. The implantation tool 12 includes a sleeve 29 mounted on the shaft 18. The sleeve 29 has a transverse peripheral circular cross section for snug sliding displacement in the blind recess 17C. The recess 17C includes a biasing member 17D in the form a compression spring, and the like, for biasing the sleeve 29 to a forward position for urging the normal open clamping jaw pair 28 into a clamping position for clamping on the segment-like carrier plate 26 (see Figure 1A and Figure 1B). The sleeve 29 includes a finger grip 29A for urging the sleeve 29 to a rearward position in the recess 17C for compressing the biasing member 17D thereby enabling the normal open clamping jaw pair 28 to revert to its normal open position for releasing the segment-like carrier plate 26 (see Figure 2A and Figure 2B).

The uppermost clamping jaw 28A includes a spaced apart pair of clamping pins 31 for insertion though the spaced apart pair of clamping throughgoing bores 27. The lowermost clamping jaw 28B includes a spaced apart pair of clamping bores 32 for receiving the spaced apart pair of clamping pins 31. The spaced apart pair of clamping pins 31 do not protrude through the spaced apart pair of clamping bores 32 so as not to damage delicate eye tissue on implantation of the implant holder 13. Releasing the clamping arrangement 16 at an implantation site in an implanted eye, leaves the implant holder 13 and the ORTI 14 at the implantation site on withdrawal of the implantation tool 12.

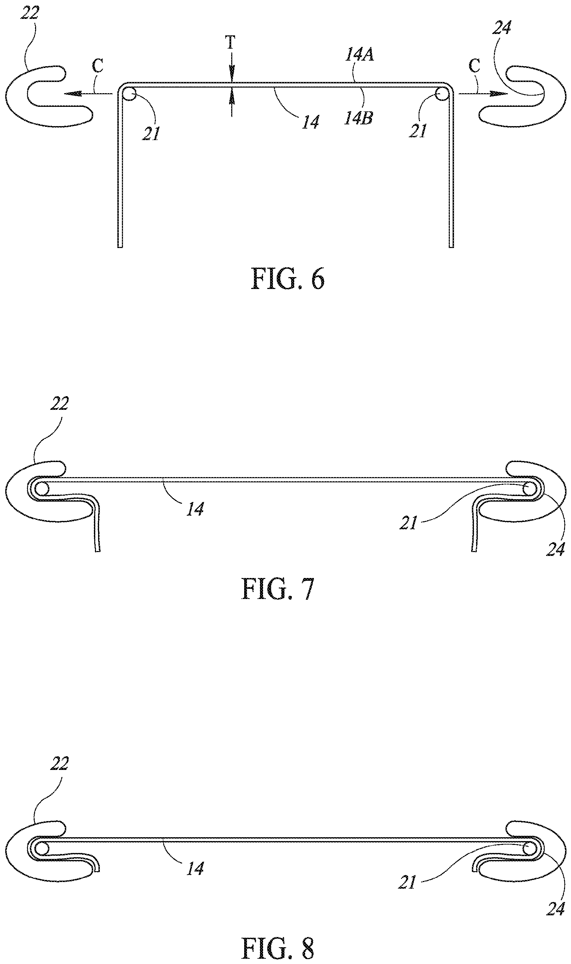

Figure 6 to Figure 8 show preparation of the implant holder 13 as follows: The ORTI 14 is laid on the implant carrier 21 with its lowermost basement membrane 14B resting thereon. The ORTI 14 is typically supplied in the same shape as the implant carrier 21 but larger such that it overhangs the implant carrier 21 to leave a downward depending edge. The ORTI 14 covers the segment-like carrier plate 26. The implant carrier 21 is compressed and is slowly released to entrap the ORTI 14 in the internal implant carrier surround groove 24 as denoted by arrows C (see Figure 6) such that the ORTI 14 is taut (see Figure 7). The ORTI 14's downward depending edge is preferably trimmed after securing in the implant holder 13 such that the implant holder 13 presents a streamline profile for implantation purposes (see Figure 8). On clamping the implant holder 13 in the implantation tool 12, the spaced apart pair of clamping pins 31 traverses the ORTI 14 overlying the segment-like carrier plate 26.

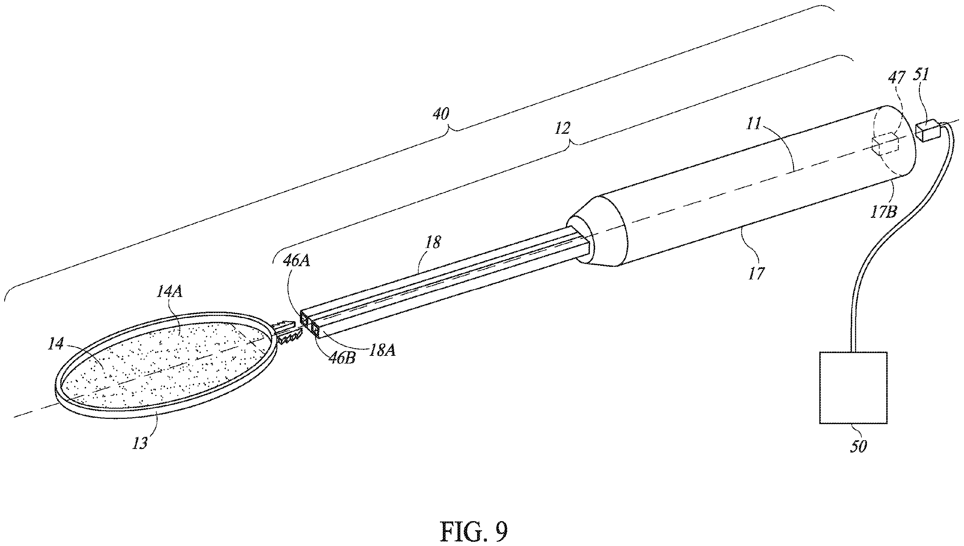

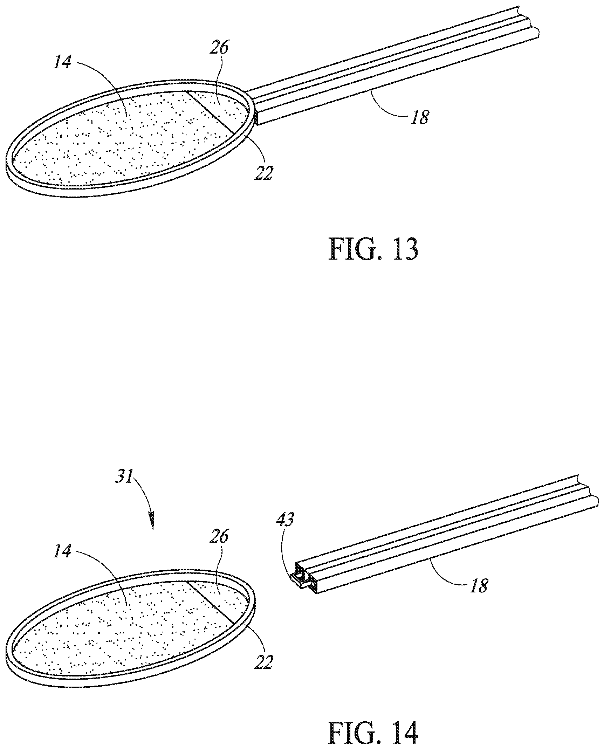

Figure 9 to Figure 14 show a handheld implantation device 40 similar to the implantation device 10 and therefore similar parts are likewise numbered. The implantation device 40 is intended for use with a user operated electrical power source 50 having an electrical plug 51. The electrical power source 50 can be operated by a user foot pedal, and the like.

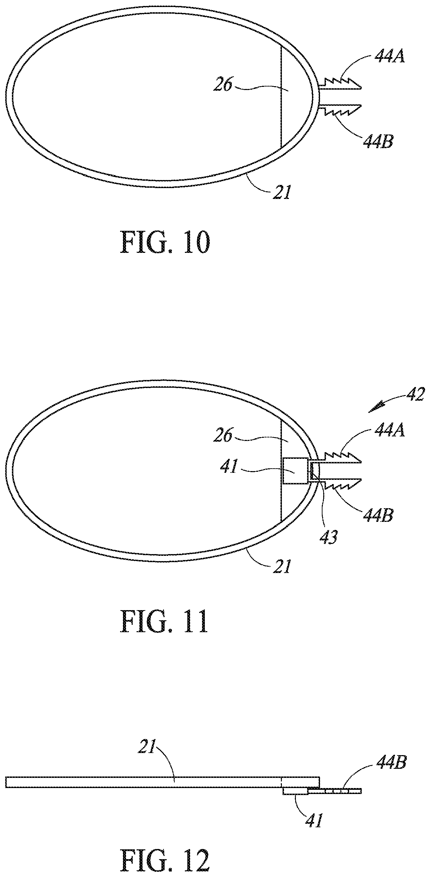

The handheld implantation device 40 includes a thermoplastic holding element 41 underlying the segment-like carrier plate 26 in the Figure 12 front elevation view such that the thermoplastic holding implant 41 faces in the same direction as the lowermost basement membrane 14B. The thermoplastic holding element 41 has a generally U-shaped electrical heating filament 42 including a crosspiece 43 and a spaced apart pair of outwardly protruding connectors 44A and 44B directed away from the leading implant holder end 13A. The crosspiece 43 can be glued or otherwise attached to the thermoplastic holding element 41. The connectors 44A and 44B are preferably barbed. The leading shaft end 18A includes a spaced apart socket pair 46A and 46B for snap fit receiving the spaced apart pair of outwardly protruding connectors 44A and 44B. The spaced apart socket pair 46A and 46B are in electrical connection with an electrical socket 47 at the trailing handle end 17B intended for receiving the electrical plug 51. The electrical heating filament 42 closes an electrical circuit with the electrical power source 50 such that, on activation of the electrical power source 50, the electrical heating filament 42 heats the thermoplastic holding element 41 whereupon the electrical heating filament 42 is detached therefrom. The electrical heating filament 42 remains inserted in the leading shaft end 18A on withdrawal of the implantation tool 12 from an implanted eye leaving the implant holder 13 with its ORTI 14 at an implantation site.

While the invention has been described with respect to a limited number of embodiments, it will be appreciated that many variations, modifications, and other applications of the invention can be made within the scope of the appended claims.

## Claims

1. A handheld implantation device (10; 40) for implantation of a retinal tissue implant at an implantation site, the handheld implantation device comprising:
(a) a handheld implantation tool (12) having a leading implantation tool end (12A) and a trailing implantation tool end (12B);
(b) an implant holder (13) for peripherally holding a retinal tissue implant (14) including an uppermost viable retinal tissue (14A) and a lowermost basement membrane (14B), and
(c) a clinician-operated attachment arrangement (16) for initial attaching said implant holder at said leading implantation tool end and subsequent selected detaching said implant holder therefrom at the implantation site for implantation of said implant holder together with the retinal tissue implant thereat.

2. The device according to claim 1 wherein said implant holder (13) includes an implant carrier (21) and a closed implant carrier surround (22) with an internal implant carrier surround groove (24) for snugly receiving said implant carrier therein for peripherally entrapping said retinal tissue implant therebetween.

3. The device according to claim 2 wherein said implant carrier (21) includes a segment-like carrier plate (26) and said lowermost basement membrane (14B) bears on said implant carrier and said segment-like carrier plate on being peripherally entrapped on said implant carrier by said closed implant carrier surround (22).

4. The device according to any one of claims 1 to 3 wherein said clinician-operated attachment arrangement (16) includes a normal open clamping jaw pair (28A, 28B) and a sleeve (29) slidable thereon between a forward position for clamping said normal open clamping jaw pair on said implant holder (13) and a rearward position for enabling said normal open clamping jaw pair to revert to its normal open position for releasing said implant holder.

5. The device according to claim 4 wherein said handheld implantation tool (12) includes a biasing member (17D) for biasing said sleeve (29) into said forward position for clamping said normal open clamping jaw pair (28A, 28B) on said implant holder (13).

6. The device according to claim 4 wherein said normal open clamping jaw pair include at least two spaced apart clamping pins (31) for passing through said implant holder (13) in said forward position.

7. The device according to any one of claims 1 to 3 for use with an electrical power source (50),
wherein said implant holder (13) includes a thermoplastic holding element (41) and an electrical heating filament (42) integrally formed with said thermoplastic holding element, said electrical heating filament being configured for insertion into said leading implantation tool end (12A) for electrical connection to said electrical power source,
whereupon, on operation of said electrical power source, said electrical power source energizes said electrical heating filament (42) for heating said thermoplastic holding element (41) thereby detaching said electrical heating filament from said thermoplastic holding element whereby said electrical heating filament remains inserted in said leading implantation tool end and said implant holder together with the retinal tissue implant (14) is detached from said leading implantation tool end at the implantation site.

## Patentansprüche

1. Tragbare Implantationsvorrichtung (10; 40) für eine Implantation eines Netzhautgewebeimplantats an einer Implantationsstelle, die tragbare Implantationsvorrichtung umfasst:
(a) ein tragbares Implantationswerkzeug (12), aufweisend ein führendes Implantationswerkzeugende (12A) und ein nachlaufendes Implantationswerkzeugende (12B);
(b) einen Implantathalter (13) zum peripheren Halten eines Netzhautgewebeimplantats (14), beinhaltend ein oberstes lebensfähiges Netzhautgewebe (14A) und eine unterste Basalmembran (14B), und
(c) eine von einem Klinikpersonal betätigte Befestigungsanordnung (16) zum initialen Anbringen des Implantathalters am führenden Implantationswerkzeugende und zum anschließenden lösbaren Abnehmen des Implantathalters davon an der Implantationsstelle zur Implantation des Implantathalters zusammen mit dem Netzhautgewebeimplantat an dieser Stelle.

2. Vorrichtung nach Anspruch 1, bei welcher der Implantathalter (13) einen Implantatträger (21) und eine geschlossene Implantatträgereinfassung (22) mit einer inneren Implantatträgereinfassungsrille (24) zur passgenauen Aufnahme des Implantatträgers darin zum peripheren Einfassen des Netzhautgewebeimplantats dazwischen enthält.

3. Vorrichtung nach Anspruch 2, bei welcher der Implantatträger (21) eine segmentartige Trägerplatte (26) beinhaltet und die unterste Basalmembran (14B) an dem Implantatträger anliegt und die segmentartige Trägerplatte durch die geschlossene Implantatträgereinfassung (22) peripher an dem Implantatträger eingefasst ist.

4. Vorrichtung nach einem der Ansprüche 1 bis 3, bei welcher die von einem Klinikpersonal betätigte Betätigungsvorrichtung (16) ein normal geöffnetes Klemmbackenpaar (28A, 28B) und eine darauf verschiebbare Hülse (29) zwischen einer Vorwärtsposition zum Klemmen des normal geöffneten Klemmbackenpaares an dem Implantathalter (13) und einer Rückwärtsposition zum Ermöglichen der Rückkehr des normal geöffneten Klemmbackenpaares in seine normale offene Position zur Freigabe des Implantathalters umfasst.

5. Vorrichtung nach Anspruch 4, bei welcher das tragbare Implantationswerkzeug (12) ein Vorspannglied (17D) zum Vorspannen der Hülse (29) in die Vorwärtsposition zum Festklemmen des normal geöffneten Klemmbackenpaares (28A, 28B) an dem Implantathalter (13) beinhaltet.

6. Vorrichtung nach Anspruch 4, bei welcher das normal geöffnete Klemmbackenpaar mindestens zwei voneinander beabstandete Klemmbolzen (31) zum Durchführen durch den Implantathalter (13) in der Vorwärtsposition beinhaltet.

7. Vorrichtung nach einem der Ansprüche 1 bis 3 zur Verwendung mit einer Elektroleistungsquelle (50),
bei welcher der Implantathalter (13) ein thermoplastisches Halteelement (41) und einen Elektroheizfaden (42), einstückig ausgebildet mit dem thermoplastischen Halteelement, beinhaltet, wobei der Elektroheizfaden eingerichtet, um in das führende Implantationswerkzeugende (12A) zur elektrischen Verbindung mit der Elektroleistungsquelle eingeführt zu sein/werden,
worauf, bei Betätigung der Elektroleistungsquelle, die Elektroleistungsquelle den Elektroheizfaden (42) zum Erwärmen des thermoplastischen Halteelements (41) energetisiert, wodurch der Elektroheizfaden von dem thermoplastischen Halteelement gelöst wird, wobei der Elektroheizfaden in dem führenden Implantationswerkzeugende eingesetzt bleibt und der Implantathalter zusammen mit dem Netzhautgewebeimplantat (14) von dem führenden Implantationswerkzeugende an der Implantationsstelle gelöst wird.

## Revendications

1. Dispositif d'implantation portatif (10 ; 40) pour l'implantation d'un implant de tissu rétinien sur un site d'implantation, le dispositif d'implantation portatif comprenant :
(a) un outil d'implantation portatif (12) ayant une extrémité avant d'outil d'implantation (12A) et une extrémité arrière d'outil d'implantation (12B) ;
(b) un support d'implant (13) pour maintenir de manière périphérique un implant de tissu rétinien (14) comprenant un tissu rétinien viable le plus haut (14A) et une membrane basale la plus basse (14B), et
(c) un agencement de fixation actionné par un clinicien (16) pour fixer initialement ledit support d'implant à ladite extrémité avant d'outil d'implantation, puis à détacher de manière sélective ledit support d'implant de celle-ci sur le site d'implantation pour implantation dudit support d'implant avec l'implant de tissu rétinien à cet endroit.

2. Dispositif selon la revendication 1, dans lequel ledit support d'implant (13) comprend un porte-implant (21) et une périphérie fermée de porte-implant (22) avec une rainure interne (24) de périphérie de porte-implant pour recevoir de manière ajustée ledit porte-implant dans celle-ci pour piéger entre eux en périphérie ledit implant de tissu rétinien.

3. Dispositif selon la revendication 2, dans lequel ledit porte-implant (21) comprend une plaque porteuse en forme de segment (26) et ladite membrane basale la plus basse (14B) appuie contre ledit porte-implant et ladite plaque porteuse en forme de segment (26) étant piégée de manière périphérique sur ledit porte implant par ladite périphérie fermée de porte-implant (22).

4. Dispositif selon l'une quelconque des revendications 1 à 3, dans lequel ledit agencement de fixation (16) actionné par un clinicien comprend une paire de mâchoires de serrage normalement ouvertes (28A, 28B) et un manchon (29) pouvant coulisser sur celles-ci entre une position avant pour serrer ladite paire de mâchoires de serrage normalement ouvertes sur ledit support d'implant (13) et une position arrière pour permettre à ladite paire de mâchoires de serrage normalement ouvertes de revenir à sa position normalement ouverte pour libérer ledit support d'implant.

5. Dispositif selon la revendication 4, dans lequel ledit outil d'implantation portatif (12) comprend un élément de sollicitation (17D) pour solliciter ledit manchon (29) vers ladite position avant pour serrer ladite paire de mâchoires de serrage normalement ouvertes (28A, 28B) sur ledit support d'implant (13).

6. Dispositif selon la revendication 4, dans lequel ladite paire de mâchoires de serrage normalement ouvertes comprend au moins deux broches de serrage (31) espacées l'une de l'autre pour traverser ledit support d'implant (13) dans ladite position avant.

7. Dispositif selon l'une quelconque des revendications 1 à 3, destiné à être utilisé avec une source d'énergie électrique (50),
dans lequel ledit support d'implant (13) comprend un élément de maintien thermoplastique (41) et un filament de chauffage électrique (42) formé intégralement avec ledit élément de maintien thermoplastique, ledit filament chauffant électrique étant configuré pour être inséré dans ladite extrémité avant d'outil d'implantation (12A) pour une connexion électrique à ladite source d'énergie électrique,
à la suite de quoi, lors du fonctionnement de ladite source d'énergie électrique, ladite source d'énergie électrique alimente ledit filament chauffant électrique (42) pour chauffer ledit élément de maintien thermoplastique (41), détachant ainsi ledit filament chauffant électrique (42) de l'élément de maintien thermoplastique, ledit filament chauffant électrique restant inséré dans ladite extrémité avant d'outil d'implantation et ledit support d'implant avec l'implant de tissu rétinien (14) étant détaché de ladite extrémité avant d'outil d'implantation au niveau du site d'implantation.
